# EUROPEAN PATENT SPECIFICATION

(11) **EP 1 991 181 B1**
(45) Date of publication and mention of the grant of the patent: **06.06.2012**
(21) Application number: 06728437.2
(22) Date of filing: 20.02.2006
(51) Int. Cl.: A61F 5/14, A43B 13/18

(54) **INSOLE**
EINLEGESOHLE
SEMELLE ORTHOPÉDIQUE

(43) Date of publication of application: 19.11.2008
(73) Proprietor: Fusco, Maria Antonietta, 83100 Avellino (IT)
(72) Inventor: Fusco, Maria Antonietta, 83100 Avellino (IT)
(74) Representative: Papa, Elisabetta
(86) International application number: PCT/IT2006/000087
(87) International publication number: WO 2007/096907

(56) References cited:
- EP-A- 1 557 105
- CA-A1- 2 048 240
- FR-A1- 2 769 801
- JP-A- 9 056 413
- US-A- 4 020 570
- US-A- 6 024 575

## Description

The present invention relates to an orthopedic plantar, in particular of the kind suitable for reflexological stimulation of the sole of the foot.

Several orthopedic plantars are known, integrally made with compliant and soft polyurethane-type materials. In particular, a type of material commonly used for making said plantars is ethylene-vinyl-acetate (EVA).

The shape of said known plantars is characterized by the presence of retrocapital olives or bars for forefoot correction and of crescents onto the medial longitudinal arch, said plantars having a substantially overlappable shape when used for a flat foot as well as for a hollow foot. The abovementioned correction areas may be present in an isolated or multiple manner, yet generally always in a number no higher than two or three zones at a time. The thicknesses reached by the correction zones are remarkable, from a minimum of 1 cm to overall 3-5 cm; in addition, the so-called heel "unloading" is present, i.e. at the heel zone the plantar has a 2-3 mm thickness, actually positioning the heel lower than the other parts of the foot. A first drawback of said known plantars is that their overall dimensions are remarkable, so much so as to force the subject to use shoes one or two sizes bigger with respect to those habitually worn-on without plantar.

Moreover, suchlike plantars tend to deform permanently in relatively short times, at the expense of their effectiveness, and their elastic features are often less than satisfactory and less than adequate to the kind of application, also because they are limited to a mere two or three zones of the sole. Moreover, it has to be pointed out that during the prescription of an orthopedic plantar orthosis, usually neither the sensory effect induced thereby nor the neuromuscular reactions linked to the sensory stimulation are assessed.

US 6,024,575 discloses an insole made of ethylene-vinil-acetate, silicone or plasticized polyurethane and lined with frelene or polynylon. Nodes protrude at the upper face of the sole.

Hence, the technical problem underlying the present invention is to provide an plantar overcoming the drawbacks mentioned with reference to the known art.

Such a problem is solved by an plantar according to claim 1.

Preferred features of the present invention are present in the dependent claims thereof.

The present invention provides several relevant advantages. In particular, the selection of the specific materials and assembling proposed and the configuration of the plantar attain properties of elasticity, stability and durability higher than those of analogous known plantars. Moreover, therapeutic or anyhow stimulatory effectiveness being equal, such a selection limits the thicknesses of the plantar.

Other advantages, features and the cosmetic operation modes of the present invention will be made apparent in the following detailed description of some embodiments thereof, given by way of example and not for limitative purposes. Reference will be made to the figures of the annexed drawings, wherein:
- Figure 1 shows a plan view of a first embodiment of the plantar according to the present invention;
- Figure 1A shows a cross-sectional view of the plantar of Figure 1 taken along line A-A of the latter;
- Figure 2 shows a side view of part of the plantar of Figure 1;
- Figure 3 shows a plan view of a second embodiment of the plantar according to the present invention; and
- Figure 4 shows a plan view of a third embodiment of the plantar according to the present invention.

With reference to said figures, an orthopedic plantar apt to the reflexological stimulation of the sole according to a preferred embodiment of the invention is generally indicated by 1.

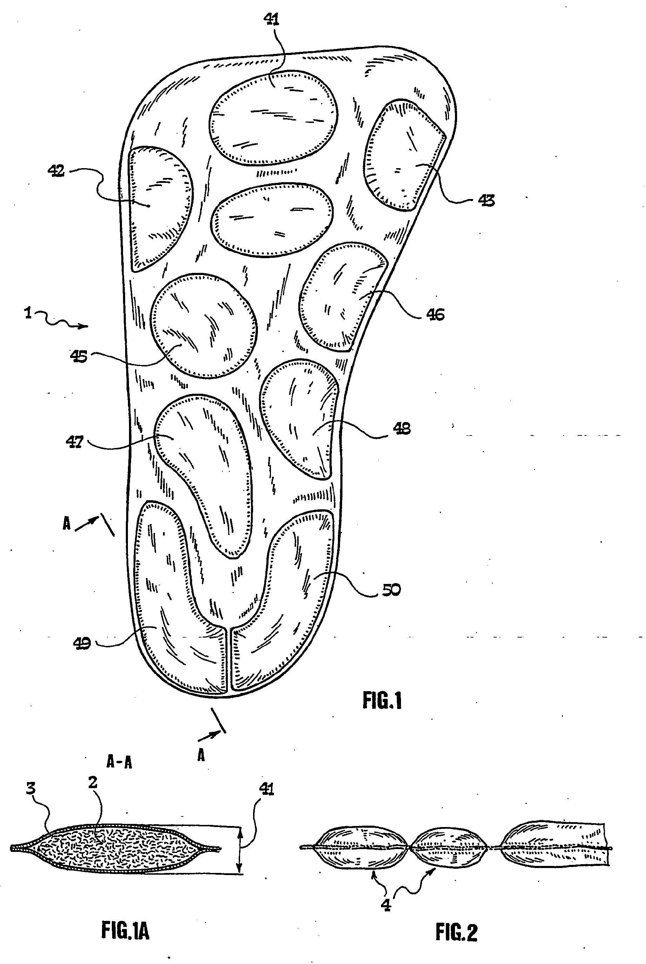

As it is apparent in Figure 1A, the plantar 1 comprises a main body 2 carrying out said stimulation, that, according to the invention, is made of a polyurethane material, and in particular of ethylene-vinyl-sulphate with a density of 50 (EVZ 50) or PLZ with a density of 29 (PLZ 29).

In particular, there can be used the material known under the trade name of Evazote 50.

Hereinafter there are reported, by way of example, some technical features of the materials preferred in the present embodiment.

| **EVZ 50** | |
|---|---|
| Composition | EVA + Expanded polyethylene |
| Cell structure | Closed |
| Color | Black/White |
| Density | 50 +/- 5 Kg/m³ |
| Water absorption (7dd.) | < 0.30% |
| Constant temperature range | MIN - 70° C/+ 70° C MAX |
| Ultimate elongation | 220% |
| Ultimate strength | 0.93 KgG/cm² (930 kPa) |
| Tearing resistance | 1.300 N/m |
| permanent set 72h, 50% compressed | 28% set |
| Compression strength | At 25% - 0.40 Kg/cm² (40 kPa) |
| Heat conductivity | 0.040 W/mK |
| Shore hardness 00 | 44 |

| **PLZ 29** | |
|---|---|
| Composition | Expanded polyethylene |
| Cell structure | Closed |
| Color | Black/White |
| Density | 29 +/- 5 Kg/m³ |
| Water absorption (7dd.) | 0.30% |
| Constant temperature range | MIN - 70° C / + 105° C MAX |
| Ultimate elongation | > 125% |
| Ultimate strength | 4 Kg/cm² (400 kPa) |
| Tearing resistance | 6.5 Kg/cm (0.65 kN/m) |
| Permanent set 72h 50% compressed | 29% set |
| Compression strength | At 25% - 0.38 Kg/cm² (38 kPa) |
| | At 50% - 1.1 Kg/cm² (110 kPa) |
| Heat conductivity | 0.039 W/mK |
| Shore hardness 00 | 53 |

In addition, the main body 2 is enveloped in a sheath in elastic material, in particular, in the present exemplary embodiment, microfiber and preferably a rovera-type fabric. Such an assembling of the plantar makes the plantar hygienic, washable and sterilizable, prevents bacterial or mycotic proliferation and, also as a consequence of this, makes it long-lasting, with no contraindication or possible damage to the subject.

The plantar 1 has, to effect said stimulation, a plurality of elastic rises or bulges of different shape and dimensions, each generally denoted by 4 in Figure 2, arranged at selected areas of the sole and specifically at the main joints of the foot. In particular, the plantar 1 provides three bulges, denoted by 41, 42 and 43, respectively, arranged at the metatarsophalangeal joints, two bulges 45 and 46 arranged at the Lisfranc's joint and two bulges 47 and 48 arranged at the Chopart's joints. As it is apparent in Figure 1, two bulges 49 and 50 involve the heel zone as well, determining therein not merely an "unloading", as it is commonly and universally the case with known orthopedic plantars, but also a stimulation and a stress allowing a greater stabilization of the Achilles tendon and the entire achilles-calcaneal system.

In fact, this structure is the one most suffering from repercussions of the knocks against resting ground during walking, as well as the one bearing all the weight of the body and on which there unloads the force of gravity against which we fight to keep the orthostasis.

Preferably, each bulge 4 has a height 41 comprised in a range of about 3-10 mm, and even more preferably equal to about 8 mm. Such a height allows to attain an ideal stimulation in terms of intensity thereof and elasticity of the support; moreover, by virtue of the type of material selected, it allows to reduce the thickness dimensions of the plantar, stimulation effectiveness being equal, to the advantage of the ease of wear. Specifically, said reduced dimensions allow the use of the plantar in any kind of shoe, and in particular in tight-fitting women's shoes, even with high heels that *per se* may cause serious foot problems to the subject wearing them.

The hereto-described bulge arrangement and configuration is particularly suitable for senile or aching foot therapy.

As it may be appreciated from Figure 2, in the present embodiment the bulges 4 equally involve the two faces, top and bottom, of the plantar 1. Hence, the configuration of the plantar is such that this is double-face, it being wearable at any one of its two faces without distinction, as right or left plantar, respectively.

Moreover, such a shape allows the patient's foot to be substantially suspended with respect to the internal surface of the shoe, generating a continuous transit of air that not only prevents odor stagnation and perspiration, but actually takes part in the continual massaging of the foot.

As illustrated also hereinafter, variant embodiments could provide the use of different multiple thicknesses for the different bulges of the plantar, rather than an uniform thickness for all the bulges as in the embodiment described hereto.

By now, it will be better appreciated that the plantar of the invention allows to optimally stress certain spots of the sole, for vascular and/or parenchymal and/or osteoarticular purposes, thereby being of wide applicability, especially in the fields of cosmetics and podiatry.

The material selected for the main body, considered *per se* and, e.g., in the form of a homogeneous small- or large-thickness lamination, exhibits features of softness to the touch, surely pleasant, yet generally not therapeutic. In particular, the Applicants verified that the polyurethane material *per se,* and in particular the abovementioned EVZ or PVZ, is absolutely inert and passive, and could indeed cause remarkable and body-harming interferences, whereas when enveloped in an elastic coating, in particular of microfiber, and advantageously compressed in well-defined cells, it expresses a compliance and a compression return elasticity optimizing the therapeutic use thereof. In particular, the preferred organization in multiple thicknesses arranged at different distances and of unequal shape and dimensions provides, said material of the main body with an elastic feature and a therapeutic value that it does not express by itself. Moreover, the plantar of the invention is particularly suitable also for cosmetic applications, in particular for the improvement of venous and lymphatic circulation, and for podiatry, in situations of metatarsalgia and foot pain from arthrosis or senile feet.

Variant embodiments may provide for the plantar to involve the entire sole, i.e. also the zone of the toes, unlike the plantar of Figure 1.

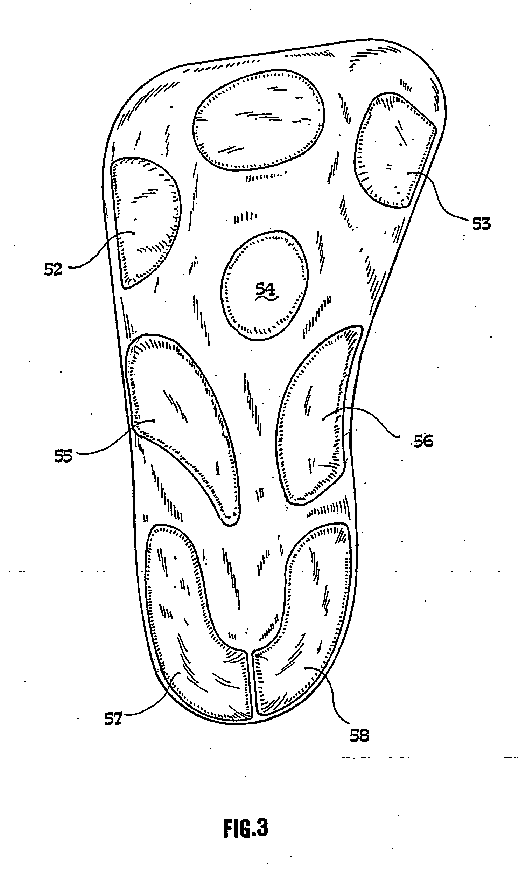

According to a variant embodiment shown in Figure 3 and particularly suitable for the above-mentioned cosmetic purposes, the arrangement of the bulges is partially different from that of the preceding embodiment. In particular, the bulges are arranged at the sole areas forming part of the so-called Lejars' venous sole (consider in particular the bulges denoted by 52, 53, 54, 55 and 56), and specifically along the longitudinal arches, both medial and lateral one. In this case as well, at the heel bone there are provided bulges (denoted by 57 and 58), in this latter zone causing the same effects already described with reference to the plantar of Figure 1. In this embodiment as well it is provided that the bulges have substantially uniform thickness, preferably equal to about 8 mm.

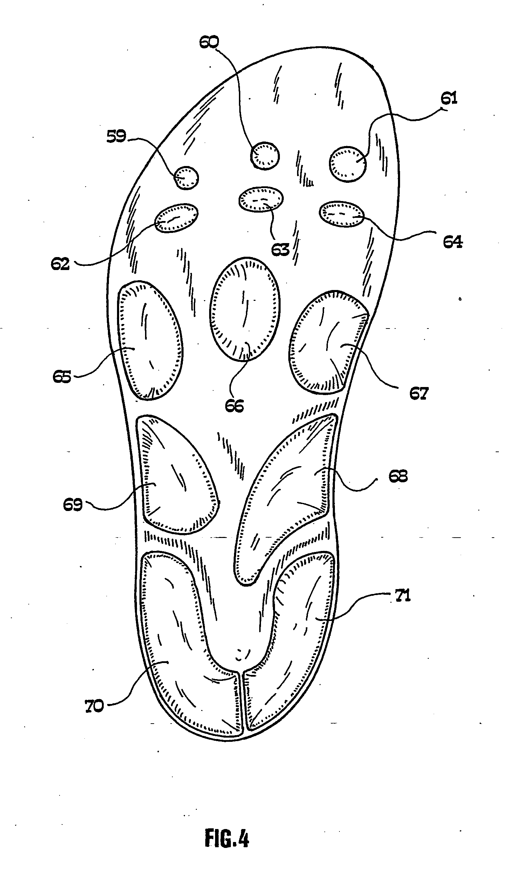

According to a third embodiment shown in Figure 4, the plantar of the invention is specifically contrived for diabetic foot care and the bulges are arranged at the arteriovenous transit zones. In particular, the plantar of this embodiment provides three bulges, 59, 60 and 61, respectively, arranged at the toe pads and preferably having a thickness of about 3 mm, three bulges, 62, 63 and 64, respectively, arranged at the interphalangeal joints and preferably having a thickness of about 4 mm, five bulges set back with respect to the preceding ones, 65, 66, 67, 68 and 69, respectively, preferably having a thickness equal to about 8 mm, and two bulges at the heel bone, 70 and 71, respectively, them also preferably having a thickness equal to about 8 mm.

Therefore, the plantar extends below the entire sole, toes included.

Said different thicknesses, at the level of the interphalangeal joints of the toes and also at the resting spots of the pads of the distal phalanxes of the toes, take into account the fact that, especially in diabetic subjects, said zones are subject to greater load and wear.

Experimental tests carried out by the Applicants highlighted that the material selected for the main body of the plantar, and in particular said materials EVZ 50 and PLZ 29, keeps its non-deformability features for a long time. A synthesis of the results of the laboratory tests carried out is reported in the annex, purely by way of example..

The present invention has hereto been described with reference to preferred embodiments thereof. It is understood that there could be other embodiments referable to the same inventive kernel, all falling within the protective scope of the claims set forth hereinafter.

### ANNEX (TEST FOR EVZ50)

### Italian Centre for Footwear Materials CIMAC

TEST REPORT: RP 20054286 Page 2 of 2

2005100306

Physico-mechanical laboratory
Tests carried out from 10.18.05 to 11.09.05

Method: EN 13520 (01) Mod. Determination of abrasion resistance of foot-contacting layer.
Test docket reference: C/04995
Test conditioning and atmosphere: 23 ± 1°C 50 ± 3% Rel. Hum.
Results:
- dry test
   At +12,800 cycles, test tubes exhibit no significant damage.
   At +6,400 cycles, test tubes exhibit no significant damage.

Method: DIN 53512 (81) -Determination of resilience
Test docket reference: C/04996 to C/04998
Test conditioning and atmosphere: 23 ± 1°C 50 ± 3% Rel. Hum.
Results:

### - Insole sample tel quel

Resilience = 35%

### - Insole sample after 7-day heat aging at 70°C

...Resilience = 32%
- Insole sample after 7-day hydrolysis treatment at 70°C/100% Rel. Hum.
...Resilience = 28%

Method: EN 20344:2004 Mod. / EN 12743 (01) - Determination of energy absorption.
Test docket reference: C/04999 to C/05001
Results:

### - Test tel quel

Energy absorption = 2.20 J

Elastic energy = 1.65 J

Energy dissipated by hysteresis = 0.55 J

### - Test after 7-day heat aging at 70°C

Energy absorption = 2.00 J

Elastic energy = 1.40 J

Energy dissipated by hysteresis = 0.60 J

### - Test after 7-day hydrolysis treatment at 70°C/100% Rel. Hum.

Energy absorption = 2.00 J

Elastic energy = 1.40 J

Energy dissipated by hysteresis = 0.60 J

### ANNEX (TEST FOR PLZ29)

### Italian Centre for Footwear Materials CIMAC

TEST REPORT: RP 20054285 Page 2 of 2

2005100305

Physico-mechanical laboratory
Tests carried out from 10.18.05 to 11.09.05

Method: EN 13520 (01) Mod. Determination of abrasion resistance of foot-contacting layer.
Test docket reference: C/04988
Test conditioning and atmosphere: 23 ± 1°C 50 ± 3% Rel. Hum.
Results:
- dry test
   At +12,800 cycles, test tubes exhibit no significant damage.
   At +6,400 cycles, test tubes exhibit no significant damage.

Method: DIN 53512 (81) - Determination of resilience
Test docket reference: C/04989 to C/04991
Test conditioning and atmosphere: 23 ± 1°C 50 ± 3% Rel. Hum.
Results:

### - Insole sample tel quel

Resilience = 30%

### - Insole sample after 7-day heat aging at 70°C

...Resilience = 28%
- Insole sample after 7-day hydrolysis treatment at 70°C/100% Rel. Hum.
...Resilience = 25%

Method: EN 20344:2004 Mod. / EN 12743 (01) - Determination of energy absorption.
Test docket reference: C/04992 to C/04994
Results:

### - Test tel quel

Energy absorption = 1.90 J

Elastic energy = 1.52 J

Energy dissipated by hysteresis = 0.38 J

### - Test after 7-day heat aging at 70°C

Energy absorption = 1.80 J

Elastic energy = 1.44 J

Energy dissipated by hysteresis = 0.36 J

### - Test after 7-day hydrolysis treatment at 70°C/100% Rel. Hum.

Energy absorption = 1.70 J

Elastic energy = 1.30 J

Energy dissipated by hysteresis = 0.40 J

## Claims

1. An orthopedic insole (1) apt to the reflexological stimulation of the sole of the foot, comprising a main body (2) carrying out said stimulation and an elastic coating (3) of said main body (2) completely enveloping it, **characterized in that** said main body (2) has a plurality of elastic bulges (4) apt to carry out said stimulation and arranged at selected areas of the sole,
**in that** said bulges (4) equally involve the two faces, top and bottom, of the insole, the insole configuration being such as to be double-face, it being wearable at any one of its two faces without distinction, as right or left insole, respectively,
and **in that** said main body (2) is made of ethylene-vinyl-sulphate (EVZ).

2. The insole (1) according to the preceding claim, wherein said main body (2) is made of EVZ with a density of 50 (EVZ 50).

3. The insole (1) according to any of the preceding claims, comprising one or more bulges (4) arrangeable at Lejars' venous sole.

4. The insole (1) according to any of the preceding claims, comprising one or more elastic bulges (4) arrangeable at the heel bone (49, 50).

5. The insole (1) according to any one of the preceding claims, comprising one or more elastic bulges (4) arrangeable at an area selected from a group comprising: metatarsophalangeal joints (41, 42, 43), Lisfranc's joint (45, 46), Chopart's joints (47, 48), toe pads (59, 60, 61) and interphalangeal joints (62, 63, 64).

6. The insole (1) according to the preceding claim, comprising one or more bulges (59, 60, 61), arrangeable at the toe pads and having a thickness equal to about 3 mm.

7. The insole (1) according to claim 5 or 6, having one or more bulges (62, 63, 64), arrangeable at the interphalangeal joints and having a thickness equal to about 4 mm.

## Patentansprüche

1. Orthopädische Einlegesohle (1), geeignet zur reflexologischen Stimulation der Fußsohle, mit einem Hauptkörper (2), der die Stimulation ausführt, und einem elastischen Überzug (3) des Hauptkörpers (2), der diesen vollständig umhüllt,
**dadurch gekennzeichnet, dass** der Hauptkörper (2) mehrere elastische Ausbauchungen (4) hat, geeignet zum Ausführen der Stimulation und angeordnet in ausgewählten Bereichen der Sohle,
dass die Ausbauchungen (4) die beiden Seiten, die obere und die untere, der Einlegesohle gleichermaßen umfassen, wobei die Einlegesohlenkonfiguration so ist, dass sie doppelseitig ist, so dass die Einlegesohle auf jeder ihrer beiden Seiten unterschiedslos tragbar ist, als rechte bzw. linke Einlegesohle, und
dass der Hauptkörper (2) aus Ehtylenvinylsulfat (EVZ) hergestellt ist.

2. Einlegesohle (1) nach dem vorhergehenden Anspruch, wobei der Hauptkörper (2) aus EVZ mit einer Dichte von 50 (EVZ 50) hergestellt ist.

3. Einlegesohle (1) nach einem der vorhergehenden Ansprüche, mit einer oder mehreren Ausbauchungen (4), die in Lejars venöser Sohle angeordnet werden können.

4. Einlegesohle (1) nach einem der vorhergehenden Ansprüche, mit einer oder mehreren elastischen Ausbauchungen (4), die an dem Fersenbein (49, 50) angeordnet werden können.

5. Einlegesohle (1) nach einem der vorhergehenden Ansprüche, mit einer oder mehreren elastischen Ausbauchungen (4), die in einem Bereich angeordnet werden können, der aus einer Gruppe ausgewählt ist, welche umfasst: metatarsophalangeale Gelenke (41, 42, 43), Lisfranc-Gelenk (45, 46), Chopart-Gelenke (47, 48), Zehenpolster (59, 60, 61) und interphalangeale Gelenke (62, 63, 64).

6. Einlegesohle (1) nach dem vorhergehenden Anspruch, mit einer oder mehreren Ausbauchungen (59, 60, 61), die an den Zehenpolstern angeordnet werden können und eine Dicke haben, die etwa 3 mm beträgt.

7. Einlegesohle (1) nach Anspruch 5 oder 6, die eine oder mehrere Ausbauchungen (62, 63, 64) hat, welche an den interphalangealen Gelenken angeordnet werden können und eine Dicke haben, die etwa 4 mm beträgt.

## Revendications

1. Semelle orthopédique (1) appropriée pour la stimulation réflexologique de la plante du pied, comprenant un corps principal (2) réalisant ladite stimulation et un revêtement élastique (3) dudit corps principal (2) l'enveloppant complètement,
**caractérisée en ce que** ledit corps principal (2) a une pluralité de renflements élastiques (4) appropriés pour réaliser ladite stimulation et disposés dans des zones sélectionnées de la plante du pied,
**en ce que** lesdits renflements (4) concernent également les deux faces, supérieure et inférieure de la semelle, la configuration de la semelle étant telle qu'elle est double-face, pouvant être portée sans distinction sur l'une quelconque de ses deux faces, en tant que semelle droite ou gauche, respectivement,
et **en ce que** ledit corps principal (2) est réalisé à partir d'éthylène-sulfate de vinyle (EVZ).

2. Semelle (1) selon la revendication précédente, dans laquelle ledit corps principal (2) est réalisé à partir de EVZ avec une densité de 50 (EVZ 50).

3. Semelle (1) selon l'une quelconque des revendications précédentes, comprenant un ou plusieurs renflements (4) pouvant être disposés sur la semelle de Lejars.

4. Semelle (1) selon l'une quelconque des revendications précédentes, comprenant un ou plusieurs renflements élastiques (4) pouvant être disposés au niveau de l'os du talon (49, 50).

5. Semelle (1) selon l'une quelconque des revendications précédentes, comprenant un ou plusieurs renflements élastiques (4) pouvant être disposés au niveau d'une zone choisie dans un groupe comprenant : les articulations métatarso-phalangiennes (41, 42, 43), l'articulation de Lisfranc (45, 46), les articulations de Chopart (47, 48), les coussinets des doigts de pied (59, 60, 61) et les articulations inter-phalangiennes (62, 63, 64).

6. Semelle (1) selon la revendication précédente, comprenant un ou plusieurs renflements (59, 60, 61) pouvant être disposés au niveau des coussinets des doigts de pied et ayant une épaisseur égale à environ 3 mm.

7. Semelle (1) selon la revendication 5 ou 6, ayant un ou plusieurs renflements (62, 63, 64) pouvant être disposés au niveau des articulations inter-phalangiennes et ayant une épaisseur égale à environ 4 mm.
